# FASCICULE DE BREVET EUROPEEN

(11) **EP 3 692 499 B1**
(45) Date de publication et mention de la délivrance du brevet: **22.09.2021**
(21) Numéro de dépôt: 18792978.1
(22) Date de dépôt: 01.10.2018
(51) Int. Cl.: G06T 7/55

(54) **PROCEDE D'IMAGERIE RADIOGRAPHIQUE, DISPOSITIF DE TRAITEMENT D'IMAGE RADIOGRAPHIQUE ET DISPOSITIF D'IMAGERIE RADIOGRAPHIQUE**
RADIOGRADISCHES BILDGEBUNGSVERFAHREN, VORRICHTUNG ZUR VERARBEITUNG VON RÖNTGENBILDERN UND RADIOGRAFISCHE BILDGEBUNGSVORRICHTUNG
RADIOGRAPHIC IMAGING METHOD, RADIOGRAPHIC IMAGE PROCESSING DEVICE, AND RADIOGRAPHIC IMAGING DEVICE

(30) Priorité: 03.10.2017 FR 1759209
(43) Date de publication de la demande: 12.08.2020
(73) Titulaire: Proteor, 21850 Saint-Apollinaire (FR)
(72) Inventeur: COLOBERT, Briac, 21000 Dijon (FR); GESBERT, Jean-Charles, 35480 Saint Malo de Phily (FR); CARRÉ, Vincent, 21000 Dijon (FR)
(74) Mandataire: Fédit-Loriot
(86) Numéro de dépôt international: PCT/FR2018/052406
(87) Numéro de publication internationale: WO 2019/069001

(56) Documents cités:
- EP-A2- 2 323 101
- Jean-Charles Gesbert: "Modélisation 3D du rachis scoliotique : fusion de données et personnaliation expérimentale", Thèse de doctorat, 28 novembre 2014 (2014-11-28), pages 1-162, XP055487234, Extrait de l'Internet: URL:http://www.theses.fr/2014REN1S051.pdf [extrait le 2018-06-22]
- CAMPBELL-KYUREGHYAN N ET AL: "The prediction of lumbar spine geometry: method development and validation", CLINICAL BIOMECHANICS, BUTTERWORTH SCIENTIFIC LTD, GUILDFORD, GB, vol. 20, no. 5, 1 juin 2005 (2005-06-01), pages 455-464, XP027795127, ISSN: 0268-0033 [extrait le 2005-06-01] cité dans la demande

## Description

L'invention concerne un procédé d'imagerie radiographique d'une structure interne en trois dimensions faisant partie d'un objet situé à l'intérieur d'un champ d'observation, un dispositif de traitement d'image radiographique et un dispositif d'imagerie radiographique.

Notamment, le procédé d'imagerie selon l'invention est adapté pour localiser un point caractéristique d'une structure interne sur une image radiographique.

Dans un mode de réalisation, le procédé d'imagerie selon l'invention est également adapté pour calculer un modèle à trois dimensions d'un objet à imager dans un champ d'observation.

Des procédés d'imagerie radiographique pour la reconstruction tridimensionnelle sont connus. A titre d'exemple, la demande de brevet français FR-A-2 810 769 décrit un procédé dans lequel on calcule la forme à trois dimensions d'un modèle représentant l'objet à partir d'un modèle générique connu a priori de l'objet. L'utilisateur doit repérer manuellement sur chacune des deux images radiographiées des repères de contrôle appartenant au dit objet, qui peuvent être des points, des segments, des droites, des arcs, des contours, des arêtes ou autres, ce qui demande à la fois de sa part des connaissances précises en anatomie pour localiser précisément les repères de contrôle, du temps et une grande précision. Après ce repérage, une position géométrique de chaque repère est déterminée dans un référentiel à trois dimensions, puis utilisée pour calculer la forme du modèle à partir du modèle générique connu. Cette importante intervention humaine au niveau du repérage manuel sur les radiographies limite en particulier la reproductibilité de la reconstruction lorsque celle-ci est réalisée par un personnel non hautement spécialisé.

Il existe des procédés d'imagerie cherchant à limiter l'intervention de spécialistes. C'est le cas du procédé décrit dans le document FR-A 2 856 170. Le procédé décrit utilise un volume de confinement de l'objet, estimé à partir d'un motif géométrique visible sur deux images, et de la position de la source. Il utilise un modèle géométrique comprenant des informations permettant d'établir, depuis un estimateur de l'objet une caractéristique géométrique pour le modèle représentant l'objet.

Le procédé décrit présente l'inconvénient de calculer la forme à trois dimensions du modèle à partir de points obtenus uniquement par traitement des images radiographiques. En cas d'identification erronée des points, le modèle est faussé.

La thèse de doctorat de Jean-Charles Gesbert: "Modélisation 3D du rachis scoliotique : fusion de données et personnaliation expérimentale", 2014, pages 1-162, divulgue la modélisation 3D du rachis scoliotique en utilisant deux radiographies 2D et une enveloppe 3D externe de l'objet.

Le brevet EP 2 323 101 A2 divulgue la fusion d'une image 3D de la forme extérieur du corps avec une reconstruction 3D d'une structure interne.

La présente invention a notamment pour but de fournir un procédé fiable dans lequel l'intervention de spécialistes est limitée. En particulier, un but de l'invention est de faciliter et améliorer l'identification des structures anatomiques internes sur une radiographie à deux dimensions. Un autre but est de proposer un procédé d'imagerie de reconstruction tridimensionnelle d'un objet fiable et précis.

A cet effet, un premier objet de l'invention concerne un procédé d'imagerie radiographique d'une structure interne en trois dimensions faisant partie d'un objet situé à l'intérieur d'un champ d'observation.

Selon l'invention, le procédé comprend :
a) recevoir des premières données d'image générées dans un référentiel de référence à trois dimensions, représentatives d'au moins une image radiographique en deux dimensions de la structure interne de l'objet,
b) recevoir des deuxièmes données d'image générées dans le référentiel de référence, représentatives d'une image en trois dimensions d'une enveloppe externe de l'objet,
c) estimer à partir des deuxièmes données d'image les coordonnées dans le référentiel de référence de premiers repères caractéristiques de la structure interne, et, optionnellement, la valeur d'au moins un paramètre caractéristique de la structure interne,
d) calculer, dans le référentiel de référence, la forme à trois dimensions d'un modèle représentant ladite structure interne à partir d'un modèle générique connu a priori de ladite structure interne, ce modèle générique comportant des points correspondants aux premiers repères, le modèle calculé étant obtenu par déformation du modèle générique de façon que ledit modèle calculé suive une forme la plus proche possible d'une isométrie du modèle générique tout en maintenant en coïncidence les points du modèle générique déformé avec les premiers repères, et optionnellement en appliquant au modèle calculé le ou les paramètres caractéristiques de la structure interne,
e) choisir au moins un deuxième repère caractéristique de la structure interne à localiser sur l'image radiographique, et pour chaque deuxième repère :
   i) identifier un point appartenant au modèle calculé à l'étape d) correspondant au deuxième repère,
   (ii) calculer les coordonnées d'une projection de ce point sur l'image radiographique,
   iii) déterminer à partir des premières données d'image les coordonnées du deuxième repère sur l'image radiographique en le recherchant dans une zone de recherche située autour des coordonnées de la projection.

L'objet mentionné ci-dessus peut comprendre notamment le rachis, le bassin, ou encore le genou d'un patient, ou plus généralement être constitué par tout ou partie du corps du patient. Dans ces différents cas, les structures internes à observer peuvent être constituées notamment par les os du patient compris dans le champ d'observation.

Le ou les repères mentionnés ci-dessus sont des repères anatomiques qui peuvent être des points, des segments, des droites, des arcs, des contours, des arêtes ou autres, ou plus généralement être constitué par tout point ou élément anatomique caractéristique du squelette du patient. Eventuellement, les premiers et deuxièmes repères peuvent être les mêmes repères.

Le ou les paramètres caractéristiques de la structure interne peuvent être des paramètres anatomiques de la structure interne, tel que par exemple des dimensions de repères anatomiques. A titre d'exemple, le tour de taille ou la hauteur séparant le tour de taille au niveau sous axillaire sont des dimensions morphologiques (mesurables sur l'enveloppe externe) permettant d'estimer une dimension anatomique de la structure interne (largeur de l'os iliaque, hauteur des vertèbres).

Le procédé selon l'invention permet une intervention réduite, voire nulle, d'un opérateur pour localiser les points anatomiques (deuxièmes repères) sur une radiographie à deux dimensions, facilitant ainsi cette localisation et la rendant plus fiable. Ceci est obtenu grâce à l'estimation de la position géométrique des premiers repères à partir des données de l'image numérique en trois dimensions de l'enveloppe externe, cette estimation permettant d'adapter un modèle générique connu a priori de la structure interne à observer, qui sert ensuite à localiser les deuxièmes repères sur une image radiographiée. Ainsi, la recherche de repères sur les radiographies 2D (en deux dimensions) est initiée par la numérisation 3D (en trois dimensions) de l'enveloppe externe de l'objet. On limite de cette manière les risques de mauvaise identification des repères sur les radiographies. Ces points sont en effet recherchés dans des zones de recherche limitées, définies autour des projections des repères sur un modèle calculé et ne sont pas définies uniquement par traitement des images radiographiques.

Autrement dit, la localisation des deuxièmes repères sur les images radiographiques utilise une image numérisée en 3D de l'objet, exploitée pour corriger un modèle générique existant. La projection de ce modèle corrigé permet de déterminer une position probable des deuxièmes repères sur une radiographie en deux dimensions. Un traitement de l'image radiographique autour de chaque position probable permet alors de localiser les deuxièmes repères de manière fiable. Ainsi, ce traitement d'image nécessite moins de ressources car il est effectué dans une zone restreinte de l'image radiographique.

On notera que l'étape c) est réalisée avant l'étape (d), l'étape (b) est réalisée avant l'étape (c) et que l'étape (a) peut être réalisée à tout moment avant l'étape (e).

Les premières et deuxièmes données d'image sont par exemple des données représentatives des coordonnées des points d'une image en 2D ou en 3D. Il peut s'agir d'un signal électrique (signal d'image).

Les deuxièmes données d'image reçues à l'étape (b) peuvent être générées par un système d'imagerie comprenant au moins un capteur optique. Ces deuxièmes données d'image peuvent avantageusement être générées au moyen d'un ou plusieurs capteurs optiques, notamment non irradiants ou ionisants, par exemple un ou plusieurs capteurs optiques d'image, convertissant un signal lumineux en un signal électrique. Le signal lumineux utilisé peut être visible ou non. A titre d'exemple, un système d'imagerie apte à mesurer une profondeur optique est utilisable, par exemple du type décrit dans le document WO 2007/043036.

Dans un mode de réalisation, au cours de l'étape (c), la position géométrique estimée de la pluralité de premiers repères peut être déterminée en procédant tout d'abord à la détermination (c)(i) des coordonnées de repères externes positionnés sur l'enveloppe externe dans un référentiel à trois dimensions, puis par l'estimation (c)(ii) des coordonnées des premiers repères dans le même référentiel à partir des repères externes.

L'étape (c)(i) peut être obtenue par calcul au moyen d'un modèle mathématique, ou encore par traitement des deuxièmes données d'image, ces repères externes étant marqués sur l'enveloppe externe. On comprendra que ce marquage est réalisé avant la génération des deuxièmes données d'image reçues à l'étape (b).

Le marquage, manuel, utilisé en cas de traitement des données d'image peut être un marquage passif. Il peut consister à positionner des repères externes dessinés ou collés sur la peau du patient, présentant par exemple une forme et/une couleur particulière ou présentant une surface réfléchissante pouvant réfléchir un flash de lumière.

Les repères externes dessinés ou collés peuvent être repérés de manière manuelle, par exemple désignés sur l'image numérisée 3D par un opérateur, ou de manière automatique par exemple par des logiciels de traitement d'image permettant de différencier des couleurs, des intensités lumineuses,.... Une intervention humaine est nécessaire dans ce cas, mais reste limitée.

Le marquage peut également être un marquage actif, tel que le positionnement d'émetteurs sur la peau du patient, de tels émetteurs pouvant éventuellement émettre un signal caractéristique de leur position apte à être détecté par le système d'imagerie permettant de numériser l'enveloppe externe. Ce signal, par exemple un signal lumineux, dans le domaine visible ou non, peut être propre à chaque marqueur et/ou être émis ponctuellement à des intervalles de temps connus ou pendant une durée connue. L'intervention humaine est alors limitée au positionnement des marqueurs sur le patient.

L'étape (c)(ii) peut notamment être mise en œuvre par calcul au moyen d'un modèle mathématique. On comprend qu'aucune intervention humaine n'est alors nécessaire lorsque l'étape (c)(i) utilise aussi un modèle mathématique, améliorant la reproductibilité de la localisation. A titre d'exemple, on peut utiliser un modèle permettant de localiser des repères internes (appartenant à la structure interne) à partir de l'enveloppe externe.

Le procédé d'imagerie selon l'invention permet ainsi de localiser de manière fiable et quasiment automatique des repères sur une ou plusieurs images radiographiques.

Les premières données d'image reçues à l'étape (a) peuvent être générées par balayage en déplaçant au moins une source radioactive dans une direction de translation non parallèle à une direction de prise de vue.

De manière générale, les deuxièmes repères sont de préférence choisis parmi des repères à localiser sur chacune des deux images radiographiques (aussi appelés stéréo correspondants), et éventuellement parmi des repères à localiser sur une seule image radiographique (aussi appelés non stéréo correspondants).

Dans un mode de réalisation particulier, au cours de l'étape (a), on reçoit des premières données d'image représentatives de deux images radiographiques enregistrées selon deux directions de prise de vue non parallèles.

La réception de premières données d'images représentatives de deux images radiographiques capturées selon deux directions de prise de vue non parallèle peut être utilisée pour la reproduction en trois dimensions de la structure interne d'un objet.

A cet effet, au cours de l'étape (e), on choisit les deuxièmes repères parmi des deuxièmes repères (stéréo correspondants) à localiser sur chacune des deux images radiographiques, et, optionnellement parmi des deuxièmes repères (non stéréo correspondants) à localiser sur une seule image radiographique. Le procédé comprend alors en outre :
f) calculer les coordonnées des deuxièmes repères dans le référentiel de référence à partir de leurs coordonnées sur au moins une des images radiographiques déterminées à l'étape (e),
g) corriger le modèle calculé à l'étape (d) en faisant coïncider des points du modèle calculé correspondant aux deuxièmes repères avec les coordonnées de ceux-ci calculés à l'étape (f).

Le modèle calculé à partir d'un modèle générique connu a priori de la structure interne est ainsi établi dans un premier temps pour localiser les deuxièmes repères de l'objet (étape (e) (iii)) sur une ou sur les deux radiographies, puis est simplement corrigé pour obtenir un modèle fiable à trois dimensions de la structure interne.

L'utilisation de deuxièmes repères stéréo correspondants, à savoir visibles sur les deux images radiographiées, permet d'améliorer la qualité du modèle corrigé. La qualité du modèle corrigé peut encore être améliorée en utilisant d'autres deuxièmes repères non stéréo correspondants. Le nombre de deuxièmes repères utilisables pour corriger le modèle pourra être choisi en fonction de la nature de la structure interne à imager. A titre d'exemple, pour une vertèbre, l'utilisation d'une dizaine de repères peut suffire. En général, on utilisera de 10 à 100 repères de contrôle.

Avantageusement, dans un mode de réalisation, les premières données d'image reçues à l'étape (a) sont générées simultanément, par balayage, en déplaçant en synchronisme, dans une même direction de translation non parallèle aux directions de prise de vues, deux sources radioactives émettant deux faisceaux de rayons ionisants respectivement dans les deux directions de prise de vue. Ceci peut permettre de générer des données d'images radiographiques dans un même référentiel à trois dimensions, par exemple après une calibration appropriée des sources radioactives.

Dans un mode de réalisation, les premières données d'image et les deuxièmes données d'image sont générées simultanément. Les premières et deuxièmes données images peuvent alors être générées dans un même référentiel à trois dimensions, par exemple après une calibration appropriée des dispositifs d'enregistrement des images. Ceci permet d'éviter de procéder à un recalage des différentes images dans le même référentiel à trois dimensions et de simplifier la mise en œuvre du procédé selon l'invention. Ce référentiel peut alors servir de référentiel de référence pour le procédé de l'invention.

Dans un autre mode de réalisation, les premières données d'image et les deuxièmes données d'image peuvent être générées dans des référentiels à trois dimensions différents. Autrement dit, chaque image ou chaque type d'image est enregistrée dans un référentiel à trois dimensions qui lui est propre. C'est par exemple le cas lorsque les images sont générées à des moments distincts. Dans ce cas, au moins une des étapes de réception (a) et (b) comprend une étape de transformation des données d'image reçues vers le référentiel de référence.

Cette étape de transformation peut par exemple être réalisée au moyen d'une matrice de transformation qui permet de passer d'un référentiel à un autre. Cette matrice de transformation peut être déterminée de sorte que des éléments communs aux premières données d'image et aux deuxièmes données d'image coïncident dans le référentiel de référence choisi. Ces éléments communs peuvent faire partie de l'image tel que, par exemple, le contour des images ou être des éléments de marquage radio-opaques de dimensions connus positionnés sur l'objet à imager avant la génération des données d'image.

Ainsi, de manière générale, les premières données d'image peuvent être générées dans un premier référentiel à trois dimensions, les deuxièmes données d'image peuvent être générées dans un deuxième référentiel à trois dimensions. Dans une variante, les premier et deuxième référentiels à trois dimensions peuvent être un seul et unique référentiel, par exemple, un seul et unique référentiel de référence. Dans une autre variante, les premier et deuxième référentiels à trois dimensions peuvent être des référentiels différents. Dans ce dernier cas, au moins une des étapes de réception (a) et (b) comprend une étape de transformation des données d'image reçues pour exprimer les premières et les deuxièmes données d'image dans un même référentiel de référence, qui peut correspondre à l'un des premier et deuxième référentiels ou qui peut être un autre référentiel choisi comme référence.

Le procédé d'imagerie selon l'invention peut en outre comprendre une étape de transmission de données, notamment vers un dispositif d'affichage. Ces données peuvent être:
- des premières et/ou des deuxièmes données d'image, permettant de visualiser sur un écran la ou les images radiographiques/3D ;
- des données représentatives des coordonnées des premiers repères,
- des données représentatives des coordonnées de chaque deuxième repère déterminé à l'étape (e)iii), ce qui permet de visualiser sur un écran la position des deuxièmes repères, notamment sur la ou les images radiographiques, et,
- le cas échéant des données représentatives du modèle corrigé calculé à l'étape (g), ce qui permet de visualiser une reconstruction en 3D de la structure interne.

La visualisation de la reconstruction en 3D pourra être séparée des autres vues.

Il est également proposé un dispositif de traitement d'image radiographique comprenant des moyens agencés pour effectuer les étapes du procédé d'imagerie selon l'invention.

Notamment, le dispositif de traitement d'image radiographique comprend :
- des moyens de réception de données d'image pour recevoir des premières données d'image générées dans un référentiel à trois dimensions représentatives d'au moins une image radiographique en deux dimensions de la structure interne de l'objet, et des deuxièmes données d'image générées dans un référentiel à trois dimensions représentatives d'une image en trois dimensions d'une enveloppe externe de l'objet
- des moyens de traitement raccordés aux moyens de réception agencés pour effectuer au moins les étapes c) à e) précédemment décrites du procédé selon l'invention,
- optionnellement des moyens de transmission, notamment vers un dispositif d'affichage.

Ce dispositif de traitement d'image peut comprendre ou être intégré dans un ou plusieurs processeurs, par exemple des microcontrôleurs, des microprocesseurs ou autres.

Les moyens de réception sont par exemple un port d'entrée, une broche d'entrée, un dispositif de connexion sans fil (par exemple utilisant la technologie « Bluetooth », « Near Field Communication » ou « WIFI») ou autre. Les moyens de traitement peuvent par exemple comprendre un cœur de processeur ou CPU (de l'anglais « Central Processing Unit »). Les moyens de transmission peuvent par exemple comprendre un port de sortie, une broche de sortie, un dispositif de connexion sans fil (par exemple utilisant la technologie « Bluetooth », « Near Field Communication » ou « WIFI») ou autre.

Les moyens de traitement peuvent également être agencés pour effectuer les étapes (f) et (g) précédemment décrites.

Le dispositif de traitement d'image peut également comprendre des moyens de mémorisation reliés aux moyens de traitement.

Il est également proposé un dispositif d'imagerie radiographique d'une structure interne en trois dimensions faisant partie d'un objet comprenant :
- un premier système d'imagerie radiographique agencé pour prendre au moins une image radiographique en deux dimensions de la structure interne de l'objet au moyen d'au moins une source radioactive et pour générer dans un référentiel à trois dimensions des premières données d'image représentatives de l'image radiographique prise,
- un deuxième système d'imagerie comprenant au moins un capteur optique, agencé pour prendre une image en trois dimensions d'une enveloppe externe de l'objet et pour générer dans un référentiel à trois dimensions des deuxièmes données d'image représentatives de cette image,
- le dispositif de traitement d'image radiographique selon l'invention connecté aux premier et deuxième systèmes d'imagerie pour recevoir les premières et deuxièmes données d'image.

Il est en outre proposé un produit programme d'ordinateur comprenant des instructions ou portions de code pour exécuter les étapes du procédé d'imagerie décrit ci-dessus lorsque ces instructions sont exécutées par un dispositif de traitement d'image radiographique, notamment celui de l'invention, comprenant notamment un processeur. Ce programme peut par exemple être stocké sur un support mémoire de type disque dur, téléchargé, ou autre.

D'autres caractéristiques et avantages de l'invention apparaîtront au cours de la description suivante d'une de ses formes de réalisation, donnée à titre d'exemple non limitatif, en référence aux dessins annexés, dans lesquels :
- la figure 1 est une vue schématique d'un dispositif d'imagerie radiographique selon une forme de réalisation de l'invention, permettant d'effectuer une prise de vue de face et une prise de vue de côté par radiographie du patient et d'effectuer une numérisation 3D de l'enveloppe externe du patient,
- la figure 2 est une représentation schématique d'une image numérisée en 3D de l'enveloppe externe du patient,
- la figure 3 est une représentation similaire à la figure 2 sur laquelle sont représentés des repères externes,
- la figure 4 est une représentation schématique d'un modèle générique en 3D d'un squelette,
- la figure 5 est une représentation schématique d'un modèle en 3D calculé du squelette du patient et de ses projections sur les images radiographiques,
- les figures 6a et 6b sont des représentations schématiques en coupe transversale montrant les déformations du modèle 3D,
- la figure 7 représente les projections des points sur une radiographie et une zone de recherche,
- la figure 8 représente un modèle en 3D reconstruit du squelette du patient.

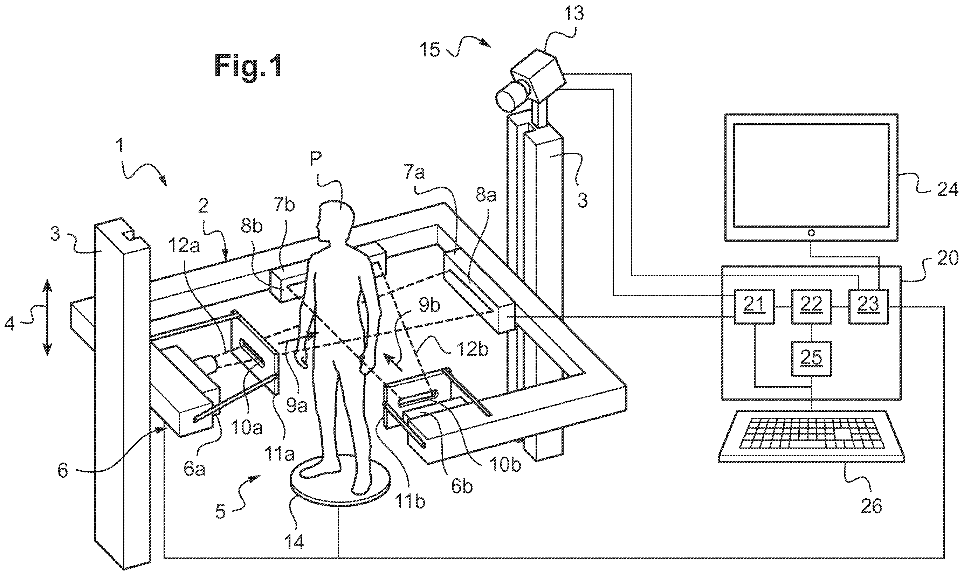

La figure 1 représente un dispositif d'imagerie radiographique 1, notamment pour la reconstruction tridimensionnelle, comportant une armature mobile 2 déplaçable verticalement de façon motorisée sur des guides verticaux 3, dans une direction de translation 4.

Cette armature entoure un champ d'observation 5 dans lequel peut prendre place un patient P, par exemple debout, ce qui permet d'observer la position des os du squelette de ce patient en station debout, ce qui peut être important par exemple pour les patients atteints de scoliose. Bien entendu, le patient pourrait être dans une position assise si nécessaire.

L'armature mobile 2 porte une première source radioactive 6a et une deuxième source radioactive 6b. Chaque source radioactive 6a, 6b est associée à un détecteur 7a, 7b, respectivement qui est disposé face à la source 6a, 6b, au-delà du champ d'observation 5. Chaque détecteur 7a, 7b comporte au moins une ligne horizontale 8a, 8b de cellules de détection. Les sources 6a, 6b et les détecteurs 7a, 7b font partie d'un premier système d'imagerie radiographique 6.

Les sources radioactives 6a, 6b sont adaptées pour émettre des rayons ionisants, notamment des rayons X, dans les directions de prise de vue 9a, 9b respectivement, ces rayonnements ionisants étant aptes à être détectés par les détecteurs 7a, 7b. La direction de prise de vue 9a est antéropostérieure par rapport au patient P alors que la direction de prise de vue 9b est latérale par rapport au patient P.

De manière classique, les rayonnements ionisants émis par les sources 6a, 6b traversent chacun une fente horizontale 10a, 10b ménagée dans un réticule 11a, 11b tel qu'une plaque métallique, pour générer un faisceau horizontal 12a, 12b de rayonnements ionisants dans le champ d'observation 5.

Bien entendu, les sources radioactives et les détecteurs pourraient être en nombre supérieur à 2, et les directions de prise de vue de ces différentes sources radioactives pourraient, le cas échéant, ne pas être perpendiculaires entre elles, ni même horizontales.

Le dispositif comprend en outre au moins un capteur optique 13 pour réaliser la numérisation externe du patient P. Tout type de capteur optique d'image est utilisable, tels que les capteurs CCD (Couple Charge Device) et CMOS (Complementary Métal Oxyde Semiconductor) ou encore les capteurs aptes à mesurer une profondeur optique. A noter cependant qu'il est préférable d'utiliser un capteur optique présentant une précision et résolution de maillage élevée.

Dans l'exemple, ce capteur optique 13 est monté sur l'un des guides verticaux 3. Le patient est en en outre debout sur un plateau 14 qui peut être mis en rotation au moyen d'un moteur. Une image en 3D du patient peut ainsi être prise en faisant tourner le plateau 14 sur 360°. Cette image 3D pourrait être prise en positionnant plusieurs capteurs optiques 13 autour du patient P au lieu de le faire tourner. 5 ou 6 capteurs optiques répartis autour du patient pourraient par exemple être utilisés pour numériser l'enveloppe externe du patient P, notamment simultanément à la prise des radiographies. Dans une autre variante, l'image 3D pourrait être prise en déplaçant un unique capteur optique 13 autour du patient, soit manuellement, soit automatiquement au moyen d'un bras motorisé, d'un rail, ou autre.

Le ou les capteurs optiques 13 font partie d'un deuxième système d'imagerie 15.

Les deux détecteurs 7a, 7b et le capteur optique 13 sont reliés à un dispositif de traitement d'image radiographique 20, par exemple un micro-ordinateur ou autre système électronique de commande tel que, par exemple un ou plusieurs processeurs de type microprocesseur, microcontrôleur ou autre.

Le micro-ordinateur 20 comporte des moyens d'exécution d'un programme d'ordinateur adapté pour mettre en œuvre le procédé décrit dans la présente invention. Il comprend ainsi des moyens de réception 21 de données d'image pour recevoir des données d'image représentatives d'une ou plusieurs images radiographiques et d'une image en trois dimensions d'une enveloppe externe de l'objet. Il comprend aussi des moyens de traitement 22 raccordés aux moyens de réception 21 agencés pour exécuter les étapes (c) à (g) précédemment décrites du procédé selon l'invention. Enfin, il comprend des moyens de transmission 23, notamment vers un dispositif d'affichage 24, ici un écran, raccordés aux moyens de traitement 22. Il peut également comporter des moyens de mémorisation 25 reliés aux moyens de traitement 22, qui peuvent être une mémoire vive ou une mémoire RAM (de l'anglais « Random Access Memory »), une EEPROM (de l'anglais « (Electrically-Erasable Programmable Read-Only Memory »), ou autre. Ces moyens de mémorisation peuvent notamment mémoriser les différents modèles et données, et éventuellement le programme d'ordinateur.

Le micro-ordinateur 20 est en outre ici équipé : d'une interface d'entrée comprenant au moins un clavier 26 et généralement une souris (non représentée), et d'une interface de sortie comprenant au moins l'écran 24 et généralement une imprimante (non représentée).

Le micro-ordinateur 20 peut également être relié aux moyens d'entraînement motorisés (non représentés) contenus dans les guides 3 et dans le plateau tournant 14, aux sources 6a, 6b et au capteur optique 13, de façon à commander d'une part le déplacement vertical de l'armature 2 et l'émission des rayonnements ionisants et d'autre part la rotation du plateau tournant 14 et le fonctionnement du capteur optique 13.

Les détecteurs 7a, 7b peuvent par exemple être des détecteurs gazeux sensibles aux basses doses de rayonnements, par exemple du type décrit dans les documents FR-A-2 749 402 ou FR-A-2 754 068. Bien entendu, d'autres types de détecteurs pourraient éventuellement être utilisés dans le cadre de la présente invention. La détection peut être réalisée par un détecteur purement linéaire (une ligne d'image à la fois), ou par un détecteur matriciel de n'importe quel rapport de forme.

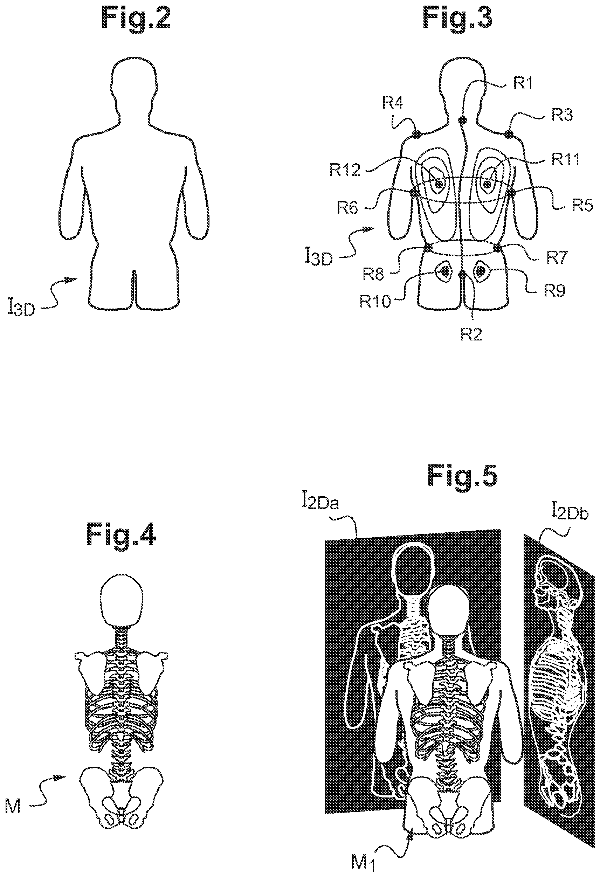

Le fonctionnement du dispositif qui vient d'être décrit est maintenant détaillé en référence aux figures 2 à 8.

Au moyen du micro-ordinateur 20, on fait prendre deux images radiographiques numériques I2Da, I2Db du patient P, par exemple en faisant balayer le champ d'observation 5 par les faisceaux 12a, 12b de rayonnements ionisants sur une hauteur correspondant à la hauteur de la zone du patient à observer, par exemple le rachis et le bassin, voire l'ensemble du squelette. A cet effet, l'armature 2 est de préférence déplaçable sur une hauteur suffisante, par exemple de 70 cm ou plus, voire de 1 mètre ou plus. Suite à une calibration appropriée des sources 6a, 6b et des détecteurs 7a, 7b, le système d'imagerie radiographique 6 génère des premières données d'image des deux radiographies dans un même référentiel à trois dimensions.

Avant ou après la prise des images radiographiques numériques, toujours au moyen du micro-ordinateur 20, on prend une image numérique en trois dimensions I3D (fig. 2) de l'enveloppe externe du patient P en faisant tourner, notamment sur 360°, le plateau 14 supportant le patient P. Le deuxième système d'imagerie 15 génère alors des deuxièmes données d'image dans un deuxième référentiel à trois dimensions différent du précédent, les prises de vues ne pouvant être simultanées. Il est alors nécessaire de transformer les premières ou les deuxièmes données d'image pour les exprimer dans un même référentiel de référence, qui peut être l'un des référentiels dans lequel sont générées les premières ou deuxièmes données d'image ou un autre référentiel. Cette transformation peut être réalisée (par les moyens de traitement 22) au moyen d'une matrice de transformation tel que précédemment décrit. Pour une prise de cette image numérique en 3D simultanée aux radiographies, au lieu d'utiliser un plateau tournant, on pourrait utiliser plusieurs capteurs optiques 13 disposés autour du patient P. Une calibration appropriées des sources 6a, 6b et du capteur optique 13 permettrait alors de générer les premières et deuxièmes données d'image dans un même référentiel à trois dimensions.

Les images numériques I2Da, I2Db, I3D de la partie examinée du patient sont transmises aux moyens de traitement 22. On peut éventuellement les enregistrer dans la mémoire du micro-ordinateur 20 et les visualiser sur l'écran 24 du micro-ordinateur. Dans l'exemple, les images radiographiques numériques I2Da, I2Db sont des images antéropostérieures et latérales (fig. 5).

On estime ensuite une position géométrique dans le référentiel de référence en 3D d'un ou plusieurs points d'intérêt (premiers repères) appartenant à la structure interne. Ces premiers repères sont caractéristiques de la structure interne.

Cette estimation passe de préférence par l'estimation de la position géométrique de repères externes, autrement dit de repères faisant partie de l'enveloppe externe. La localisation de repères externes sur l'enveloppe externe de l'objet présente l'avantage de permettre une estimation sans radiation de la structure interne de cet objet.

L'estimation de la position géométrique des repères externes est de préférence automatique, par exemple en utilisant des méthodes connues, telles que celles décrites par Michonski et al. (Automatic recognition of surface landmarks of anatomical structures of back and posture. J Biomed Opt. 2012 May;17(5):056015).

On peut aussi employer des courbures de Gauss pour identifier des repères externes qui pourront ensuite être utilisés pour estimer la position des premiers repères internes.

Tel que représenté sur la figure 3, on peut identifier préférentiellement les repères externes suivants :
R1 : vertebra prominens de C7, R2 : sommet du pli inter-fessier, R3 et R4 : épaules gauche et droite (points immédiatement à la verticale des plis sous-axillaire), R5 et R6 : plis sous-axillaires gauche et droit, R7, R8 : points gauche et droit de la taille, R9, R10 : épines iliaques postéro-supérieures gauche et droite, R11, R12 angles inférieurs de la scapula gauche et droit.

Les paramètres anatomiques tels que les dimensions des vertèbres et des structures osseuses peuvent être estimées par des équations de régressions liées à des mesures anthropométriques externes, tel que décrit par exemple par Bertrand et al., Estimation of external and internai human body dimensions from few external measurements, Journal of Musculoskeletal Research, Vol. 12, No. 4 (2009) 191-204). Une telle estimation des longueurs caractéristiques de repères anatomiques de la structure interne permet de calculer la position possible de ce repère interne (appelé ici premier repère).

Plusieurs études ont également proposé des relations externes-internes pour estimer les centres des corps vertébraux à partir de la surface externe (The prédiction of lumbar spine geometry: method development and validation. Campbell-Kyureghyan N, Jorgensen M, Burr D, Marras W. Clin Biomech (Bristol, Avon). 2005 Jun;20(5):455-64).

Différentes lois de transformation non-rigides peuvent aussi être envisagées pour estimer la position des repères de la structure interne (Seo et al. An automatic modeling of human bodies from sizing parameters. In: SI3D '03: Proceedings of the 2003 symposium on Interactive 3D graphies, New York, NY, USA, ACM Press (2003) 19-26).

Le procédé est ici présenté en faisant référence à la colonne vertébrale, mais un procédé similaire pourrait également être utilisé pour une structure interne simple dont on aurait une connaissance a priori, tel qu'un os simple, tel une seule vertèbre, une structure ligamentaire, ou autre, ou un ensemble de structures anatomiques tel qu'un membre inférieur ou supérieur, ou autres. On utilisera alors d'autres repères anatomiques adaptés aux structures à observer/imager. En outre, en fonction de la structure interne à observer/imager, la numérisation de l'enveloppe externe peut être réalisée sur le corps complet du patient ou sur une partie limitée du corps (uniquement le dos du tronc par exemple).

Dès lors que les coordonnées (dans le référentiel de référence) des premiers repères sont estimées et que les valeurs des paramètres anatomiques ont éventuellement été estimées, on peut calculer la forme à trois dimensions d'un modèle représentant la structure interne à partir d'un modèle générique de celle-ci.

A cet effet, on dispose de modèles génériques des structures internes représentés sur les images radiographiques, en particulier les vertèbres, mais éventuellement aussi d'autres structures anatomiques internes, tels les ligaments ou autres. Ces modèles peuvent être enregistrés dans le micro-ordinateur 20.

Un modèle générique, par exemple établi à partir d'une base de données, peut être défini comme un maillage de quelques centaines à quelques centaines de milliers de points d'une structure.

De tels modèles génériques sont par exemple établis à partir de bases de données contenant des données relatives à des repères particuliers de la structure. Ces données peuvent comprendre des positions de points caractéristiques de la structure, des longueurs caractéristiques de la structure, ou encore des segments, droites ou arcs caractéristiques de la structure, et/ou des contours et arêtes de la structure. Pour une vertèbre, la base contient par exemple la position d'une vingtaine de points caractéristiques de la vertèbre, des longueurs caractéristiques de la vertèbre.

La base de données peut également contenir des données relatives à l'emplacement relatif de la structure interne dans le squelette du sujet dont elle est issue. Dans le cas d'une vertèbre, il s'agit par exemple de l'orientation angulaire de la vertèbre et la courbure de la colonne au niveau de la vertèbre.

Il est possible d'établir des classements des objets dans différentes catégories afin de distinguer des données caractéristiques d'individus sains ou atteints d'une pathologie, et/ou caractéristiques du poids, de la taille, de l'âge ou de tout autre type de paramètre d'un individu.

Un modèle générique peut comporter par exemple des données statistiques (moyennes, variances, ...) pour chaque paramètre de la base de données, ou bien des équations mathématiques permettant de déterminer pour un objet donné la localisation de points caractéristiques à partir de la valeur de paramètres estimateurs de cet objet.

Dans un mode de réalisation, la forme à trois dimensions d'un modèle représentant la structure interne est calculée à partir d'un modèle générique de celle-ci par exemple en paramétrant ce dernier au moyen des premiers repères et éventuellement des paramètres anatomiques qui ont été estimées, de préférence automatiquement, à partir de l'image numérique I3D de l'enveloppe externe.

La figure 4 représente schématiquement le modèle générique M de la structure osseuse du sujet, la figure 5 montrant le modèle M1 calculé obtenu par paramétrage du modèle M. La scoliose du sujet observé est ainsi visible sur le modèle calculé M1.

Ce paramétrage du modèle générique M peut être réalisé en modifiant les dimensions du modèle générique conformément aux paramètres anatomiques.

On peut par exemple utiliser la méthode décrite par Hwang et al. ("Rapid Development of Diverse Human Body Models for Crash Simulations through Mesh Morphing," SAE Technical Paper 2016-01-1491, 2016).

Pour cela, on fait correspondre les coordonnées d'un ou plusieurs points du modèle générique M correspondants à des premiers repères avec les coordonnées de ces premiers repères estimés à partir des données de l'image I3D. Ce positionnement peut être réalisé par rapport aux positions des vertèbres associées à chaque paire de côtes. Puis, on déforme le modèle générique géométriquement en conservant les longueurs pour correspondre aux contraintes données par l'enveloppe externe. Un modèle mécanique peut également être utilisé à cette étape *(*Closkey, R. F., Schultz, A. B. and Luchies, C. W. (1992) A model for studies of the deformable rib cage. Journal of Biomechanics, 25, 529-539).

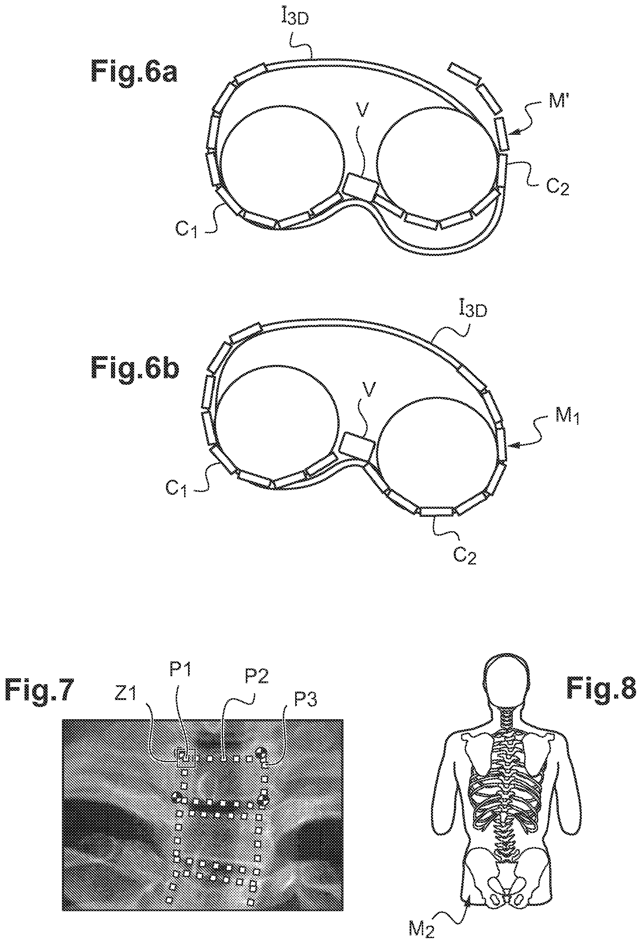

Par exemple, lorsque la structure à imager est le gril costal, le modèle générique M est d'abord mis aux dimensions du patient au moyen des premiers repères et des paramètres anatomiques pour obtenir un modèle M', puis, il est positionné à l'intérieur de l'image numérisée I3D de l'enveloppe externe par rapport aux positions des vertèbres associées à chaque paire de côtes, tel que représenté sur la figure 6a. Le modèle M' est alors dans le même référentiel de référence que l'image I3D de l'enveloppe externe. Sur cette figure 6a, la lettre « V » désigne une vertèbre, à laquelle sont reliées les côtes C1 et C2. Le trait plein correspond à l'image de l'enveloppe externe I3D, alors que les traits pointillés correspondent au modèle M'. Les côtes C1, C2 du modèle M' sont ensuite déformées géométriquement en conservant leurs longueurs pour correspondre aux contraintes données par la forme externe du tronc, en particulier les gibbosités thoraciques, tel que représenté sur la figure 6b. On obtient ainsi un modèle 3D calculé M1 de la structure interne à observer/imager. Lors de cette étape, les techniques d'animation de rigging (déformation du squelette par cinématique directe ou inverse) et de skinning (association de chaque os du squelette à une portion de la peau) peuvent être utilisées (Avatar reshaping and automatic rigging using a deformable model. Feng A., Casas D., Shapiro A.,Proceeding MIG '15 Proceedings of the 8th ACM SIGGRAPH Conférence on Motion in Games p 57-64).

On procède ensuite à la projection du modèle 3D calculé M1 sur chacune des images radiographiées I2Da, I2Db (fig.5). Cette projection permet un repérage de repères anatomiques (deuxièmes repères) dans les radiographies 2D sans nécessiter de traitement d'image, ce qui permet d'accélérer ce repérage et de le rendre plus fiable. Le choix des deuxièmes repères peut être automatique ou manuel avec une désignation d'un opérateur. Ces deuxièmes repères peuvent être les mêmes que les premiers repères ou non.

La projection utilisée est par exemple une projection linéaire. Cette projection peut être effectuée en utilisant la technique DLT (acronyme anglais de « Direct Linear Transformation »- transformation linéaire directe). Il est également possible de modifier les équations de la technique DLT en procédant à une simplification. Par projection du modèle M1, on obtient ainsi les coordonnées en 2D estimées des deuxièmes repères dans chaque image radiographiée I2Da, I2Db. Cette estimation initiale est ensuite affinée (étape (e)(iii)), en définissant une zone de recherche située autour de la projection du deuxième repère du modèle calculé dans l'image radiographiée. La figure 7 représente les projections d'une pluralité de ces points Pi sur une radiographie, ou i est un nombre entier représentant un nombre de points. Pour des raisons de clarté, seuls quelques points P1, P2, P3 sont représentés. La zone Z1 correspond à la zone de recherche du repère de projection P1.

Cette étape commence par exemple par une segmentation des ROI (Region Of Interest- regions d'intérêt).

On peut alors appliquer des filtres permettant de repérer les zones de fortes densités négatives et positives (filtre orienté). Un filtre de Canny appliqué à ces ROI permet ensuite de déterminer des contours qui peuvent être utilisés par la technique de transformée généralisé de Hough (utilisant un modèle de vertèbre).

En variante, au lieu d'utiliser les filtres orientés et de Canny, l'amélioration des estimations initiales peut se faire automatiquement par une méthode de contour actif comme le flux de vecteur gradient (GVF) (Moura et al., Fast 3D reconstruction of the spine from biplanar radiographs using a deformable articulated model. Med Eng Phys. 2011 Oct;33(8):924-33).

Lorsque les projections 2D du modèle 3D M1 ont été optimisées en exploitant les données radiographiques, le modèle 3D M1 peut être reconstruit. A cet effet, on corrige par exemple le modèle M1 par Transformation Linéaire Directe (DLT) en utilisant les mêmes couples de points (avec leurs coordonnées actualisées) que lors de la projection (deuxièmes repères). Un modèle reconstruit M2 est alors obtenu (fig. 8).

Cette reconstruction est réalisée en utilisant des deuxièmes repères qui sont stéréocorrespondants. La géométrie épipolaire, utilisant des repères non stéréo correspondants peut être aussi utilisée pour obtenir une reconstruction plus précise du modèle M2 (méthode de type NSCP (Non Stereo Correspondent Point)). Par exemple, après la reconstruction des points stéréo-correspondants, le modèle M2 est initialisé. Les points non-stéréo-correspondants du modèle M2 seront projetés uniquement dans une des deux vues radiographiques (I2Da ou ID2b) dans laquelle on sait que la recherche sera possible. Le modèle M2 est réactualisé par l'intégration des points non-stéréo-correspondants.

La présente invention permet ainsi de reconstruire un modèle 3D précis d'une structure interne en limitant la dose de rayonnement émise vers le champ d'observation.

## Revendications

1. Procédé d'imagerie radiographique d'une structure interne en trois dimensions faisant partie d'un objet situé à l'intérieur d'un champ d'observation, le procédé comprenant :
a) recevoir des premières données d'image générées dans un référentiel de référence à trois dimensions, représentatives d'au moins une image radiographique en deux dimensions de la structure interne de l'objet,
b) recevoir des deuxièmes données d'image générées dans le référentiel de référence, représentatives d'une image en trois dimensions d'une enveloppe externe de l'objet, **caractérisé en ce qu'**il comprend les étapes consistant à :
c) estimer à partir des deuxièmes données d'image les coordonnées dans le référentiel de référence de premiers repères caractéristiques de la structure interne, et, optionnellement, la valeur d'au moins un paramètre caractéristique de la structure interne,
d) calculer, dans le référentiel de référence, la forme à trois dimensions d'un modèle représentant ladite structure interne à partir d'un modèle générique connu a priori de ladite structure interne, ce modèle générique comportant des points correspondants aux premiers repères, le modèle calculé étant obtenu par déformation du modèle générique de façon que ledit modèle calculé suive une forme la plus proche possible d'une isométrie du modèle générique tout en maintenant en coïncidence les points du modèle générique déformé avec les premiers repères, et optionnellement en appliquant au modèle calculé le ou les paramètres caractéristiques de la structure interne,
e) choisir au moins un deuxième repère caractéristique de la structure interne à localiser sur l'image radiographique, et pour chaque deuxième repère :
i) identifier un point appartenant au modèle calculé à l'étape d) correspondant au deuxième repère,
(ii) calculer les coordonnées d'une projection de ce point sur l'image radiographique,
iii) déterminer à partir des premières données d'image les coordonnées du deuxième repère sur l'image radiographique en le recherchant dans une zone de recherche située autour des coordonnées de la projection.

2. Procédé d'imagerie radiographique d'une structure interne en trois dimensions faisant partie d'un objet situé à l'intérieur d'un champ d'observation, le procédé comprenant :
a) recevoir des premières données d'image générées dans un référentiel à trois dimensions, représentatives d'au moins une image radiographique en deux dimensions de la structure interne de l'objet,
b) recevoir des deuxièmes données d'image générées dans un autre référentiel à trois dimensions, représentatives d'une image en trois dimensions d'une enveloppe externe de l'objet,
dans lequel au moins une des étapes de réception (a) et (b) comprend une étape de transformation des données d'image reçues pour exprimer les premières et les deuxièmes données d'image dans un même référentiel de référence, **caractérisé en ce qu'**il comprend les étapes consistant à :
c) estimer à partir des deuxièmes données d'image les coordonnées dans le référentiel de référence de premiers repères caractéristiques de la structure interne, et, optionnellement, la valeur d'au moins un paramètre caractéristique de la structure interne,
d) calculer, dans le référentiel de référence, la forme à trois dimensions d'un modèle représentant ladite structure interne à partir d'un modèle générique connu a priori de ladite structure interne, ce modèle générique comportant des points correspondants aux premiers repères, le modèle calculé étant obtenu par déformation du modèle générique de façon que ledit modèle calculé suive une forme la plus proche possible d'une isométrie du modèle générique tout en maintenant en coïncidence les points du modèle générique déformé avec les premiers repères, et optionnellement en appliquant au modèle calculé le ou les paramètres caractéristiques de la structure interne,
e) choisir au moins un deuxième repère caractéristique de la structure interne à localiser sur l'image radiographique, et pour chaque deuxième repère :
i) identifier un point appartenant au modèle calculé à l'étape d) correspondant au deuxième repère,
(ii) calculer les coordonnées d'une projection de ce point sur l'image radiographique,
iii) déterminer à partir des premières données d'image les coordonnées du deuxième repère sur l'image radiographique en le recherchant dans une zone de recherche située autour des coordonnées de la projection.

3. Procédé d'imagerie selon la revendication 1 ou 2, dans lequel, les deuxièmes données d'image reçues à l'étape b) sont générées par un système d'imagerie comprenant au moins un capteur optique.

4. Procédé d'imagerie selon l'une quelconque des revendications 1 à 3, dans lequel, l'étape c) comprend :
(i) la détermination des coordonnées de repères externes positionnés sur l'enveloppe externe dans le référentiel de référence, obtenue :
par calcul au moyen d'un modèle mathématique, ou
par traitement des deuxièmes données d'image, ces repères externes étant marqués sur l'enveloppe externe,
(ii) l'estimation des coordonnées des premiers repères dans le même référentiel à partir des repères externes.

5. Procédé d'imagerie selon l'une quelconque des revendications 1 à 4, dans lequel, les premières données d'image reçues à l'étape (a) sont générées par balayage en déplaçant au moins une source radioactive dans une direction de translation non parallèle à une direction de prise de vue.

6. Procédé d'imagerie selon l'une quelconque des revendications précédentes, dans lequel, au cours de l'étape a), on reçoit des premières données d'image représentatives de deux images radiographiques enregistrées selon deux directions de prise de vue non parallèles.

7. Procédé d'imagerie selon la revendication 6, dans lequel, au cours de l'étape e), on choisit les deuxièmes repères parmi des deuxièmes repères à localiser sur chacune des deux images radiographiques, et optionnellement, des deuxièmes repères à localiser sur une seule image radiographique,
le procédé comprenant en outre :
f) calculer les coordonnées des deuxièmes repères dans le référentiel de référence à partir de leurs coordonnées sur au moins une des images radiographiques déterminées à l'étape e),
g) corriger le modèle calculé à l'étape d) en faisant coïncider des points du modèle calculé correspondants aux deuxièmes repères avec les coordonnées de ceux-ci calculés à l'étape f).

8. Procédé d'imagerie selon la revendication 7, dans lequel les premières données d'image reçues à l'étape a) sont générées simultanément, notamment dans un même référentiel à trois dimensions, par balayage, en déplaçant en synchronisme, dans une même direction de translation non parallèle aux directions de prise de vues, deux sources radioactives émettant deux faisceaux de rayons ionisants respectivement dans les deux directions de prise de vue.

9. Procédé d'imagerie selon l'une quelconque des revendications 1 ou 3 à 8 lorsqu'elles ne dépendent pas de la revendication 2, dans lequel les premières données d'image et les deuxièmes données d'image sont générées simultanément, notamment dans un même référentiel à trois dimensions.

10. Procédé d'imagerie selon l'une quelconque des revendications 1 à 9 comprenant en outre :
une étape de transmission, notamment vers un dispositif d'affichage, des données choisies parmi les premières données d'image, les deuxièmes données d'image, des données représentatives des coordonnées des premiers repères, des données représentatives des coordonnées de chaque deuxième repère déterminé à l'étape e)iii), et, le cas échéant des données représentatives du modèle corrigé calculé à l'étape g).

11. Dispositif de traitement d'image radiographique (20) comprenant :
- des moyens (21) de réception de données d'image pour recevoir des premières données d'image générées dans un référentiel à trois dimensions, représentatives d'au moins une image radiographique en deux dimensions de la structure interne de l'objet, et des deuxièmes données d'image générées dans un référentiel à trois dimensions représentatives d'une image en trois dimensions d'une enveloppe externe de l'objet,
- des moyens (22) de traitement raccordés aux moyens de réception agencés pour effectuer au moins les étapes c) à e) du procédé selon l'une quelconque des revendications 1 à 10,
- optionnellement des moyens de transmission (23), notamment vers un dispositif d'affichage (24).

12. Dispositif d'imagerie radiographique (1) d'une structure interne en trois dimensions faisant partie d'un objet comprenant :
- un premier système d'imagerie radiographique (6) agencé pour prendre au moins une image radiographique en deux dimensions de la structure interne de l'objet au moyen d'au moins une source radioactive (6a, 6b) et pour générer dans un référentiel à trois dimensions des premières données d'image représentatives de l'image radiographique prise,
- un deuxième système d'imagerie (15) comprenant au moins un capteur optique (13), agencé pour prendre une image en trois dimensions d'une enveloppe externe de l'objet et pour générer dans un référentiel à trois dimensions des deuxièmes données d'image représentatives de cette image,
- le dispositif de traitement d'image radiographique (20) selon la revendication 11 connecté aux premier et deuxième systèmes d'imagerie pour recevoir les premières et deuxièmes données d'image.

13. Programme d'ordinateur comprenant des portions de code de programme pour l'exécution des étapes du procédé d'imagerie selon l'une quelconque des revendications 1 à 10, lorsque ledit programme est exécuté par un dispositif de traitement d'image radiographique (20).

## Patentansprüche

1. Radiografisches Bildgebungsverfahren einer internen Struktur in drei Dimensionen, das Teil eines Gegenstandes ist, der im Inneren eines Beobachtungsfeldes gelegen ist, wobei das Verfahren umfasst:
a) Empfangen erster Bilddaten, die in einem Referenzbezugssystem mit drei Dimensionen erzeugt werden, die repräsentativ für mindestens ein radiografisches Bild in zwei Dimensionen der internen Struktur des Gegenstandes sind,
b) Empfangen zweiter Bilddaten, die in dem Referenzbezugssystem erzeugt werden, die repräsentativ für ein Bild in drei Dimensionen einer externen Hülle des Gegenstandes sind,
**dadurch gekennzeichnet, dass** es die Schritte umfasst, bestehend aus:
c) Schätzen aus den zweiten Bilddaten der Koordinaten in dem Referenzbezugssystem von ersten charakteristischen Bezugspunkten der internen Struktur, und optional des Wertes mindestens eines charakteristischen Parameters der internen Struktur,
d) Berechnen, in dem Referenzbezugssystem, der Form in drei Dimensionen eines Modells, das die interne Struktur darstellt, aus einem zuvor bekannten generischen Modell der internen Struktur, wobei dieses generische Modell Punkte beinhaltet, die den ersten Bezugspunkten entsprechen, wobei das berechnete Modell durch Verfomen des generischen Modells derart erhalten wird, dass das berechnete Modell einer möglichst nächstgelegenen Form einer Isometrie des generischen Modells folgt, und dabei die Punkte des verformten generischen Modells mit den ersten Bezugspunkten in Überdeckung hält, und optional durch Anwenden auf das berechnete Modell des oder der charakteristischen Parameter der internen Struktur,
e) Wählen mindestens eines zweiten charakteristischen Bezugspunktes der zu lokalisierenden internen Struktur auf dem radiografischen Bild, und für jeden zweiten Bezugspunkt:
i) Identifizieren eines Punktes, der dem im Schritt d) berechneten Modell angehört, entsprechend dem zweiten Bezugspunkt,
(ii) Berechnen der Koordinaten einer Projektion dieses Punktes auf dem radiografischen Bild,
iii) Bestimmen aus den ersten Bilddaten der Koordinaten des zweiten Bezugspunktes auf dem radiografischen Bild durch dessen Suche in einer Suchzone, die um die Koordinaten der Projektion herum gelegen ist.

2. Radiografisches Bildgebungsverfahren einer internen Struktur in drei Dimensionen, das Teil eines Gegenstandes ist, der im Inneren eines Beobachtungsfeldes gelegen ist, wobei das Verfahren umfasst:
a) Empfangen der ersten Bilddaten, die in einem Bezugssystem mit drei Dimensionen erzeugt werden, die repräsentativ für mindestens ein radiografisches Bild in zwei Dimensionen der internen Struktur des Gegenstandes sind,
b) Empfangen der zweiten Bilddaten, die in einem weiteren Bezugssystem mit drei Dimensionen erzeugt werden, die repräsentativ für ein Bild in drei Dimensionen einer externen Hülle des Gegenstandes sind, wobei mindestens einer der Schritte des Empfangens (a) und (b) einen Transformationsschritt der empfangenen Bilddaten umfasst, um die ersten und die zweiten Bilddaten in einem selben Referenzbezugssystem auszudrücken,
**dadurch gekennzeichnet, dass** es die Schritte umfasst, bestehend aus:
c) Schätzen aus den zweiten Bilddaten der Koordinaten in dem Referenzbezugssystem von ersten charakteristischen Bezugspunkten der internen Struktur, und optional des Wertes mindestens eines charakteristischen Parameters der internen Struktur,
d) Berechnen in dem Referenzbezugssystem der Form in drei Dimensionen eines Modells, das die interne Struktur darstellt, aus einem zuvor bekannten generischen Modell der internen Struktur, wobei dieses generische Modell Punkte beinhaltet, die den ersten Bezugspunkten entsprechen, wobei das berechnete Modell durch Verfomen des generischen Modells derart erhalten wird, dass das berechnete Modell einer möglichst nächstgelegenen Form einer Isometrie des generischen Modells folgt, und dabei die Punkte des verformten generischen Modells mit den ersten Bezugspunkten in Überdeckung hält, und optional durch Anwenden auf das berechnete Modell des oder der charakteristischen Parameter der internen Struktur,
e) Auswählen mindestens eines zweiten charakteristischen Bezugspunktes der zu lokalisierenden internen Struktur auf dem radiografischen Bild, und für jeden zweiten Bezugspunkt:
i) Identifizieren eines Punktes, der dem im Schritt d) berechneten Modell angehört, entsprechend dem zweiten Bezugspunkt,
(ii) Berechnen der Koordinaten einer Projektion dieses Punktes auf dem radiografischen Bild,
iii) Bestimmen aus den ersten Bilddaten der Koordinaten des zweiten Bezugspunktes auf dem radiografischen Bild durch dessen Suche in einer Suchzone, die um die Koordinaten der Projektion herum gelegen ist.

3. Bildgebungsverfahren nach Anspruch 1 oder 2, wobei die im Schritt b) empfangenen zweiten Bilddaten durch ein Bildgebungssystem erzeugt werden, das mindestens einen optischen Sensor umfasst.

4. Bildgebungsverfahren nach einem der Ansprüche 1 bis 3, wobei der Schritt c) umfasst:
(i) das Bestimmen der Koordinaten von externen Bezugspunkten, die auf der externen Hülle in dem Referenzbezugssystem gelegen sind, erhalten:
durch Berechnen anhand eines mathematischen Modells, oder
durch Verarbeiten der zweiten Bilddaten, wobei diese externen Bezugspunkte auf der externen Hülle markiert sind,
(ii) das Schätzen der Koordinaten der ersten Bezugspunkte in demselben Bezugssystem aus den externen Bezugspunkten.

5. Bildgebungsverfahren nach einem der Ansprüche 1 bis 4, wobei die ersten in Schritt a) empfangenen Bilddaten durch Abtasten durch Verschieben mindestens einer radioaktiven Quelle in einer Vorschubrichtung nicht parallel zu einer Bildaufnahmerichtung erzeugt werden.

6. Bildgebungsverfahren nach einem der vorstehenden Ansprüche, wobei im Laufe des Schrittes a) erste repräsentative Bilddaten von zwei in zwei nicht parallelen Aufnahmerichtungen aufgezeichneten radiografischen Bildern empfangen werden.

7. Bildgebungsverfahren nach Anspruch 6, wobei im Laufe des Schrittes e) zwei Bezugspunkte aus den zweiten zu lokalisierenden Bezugspunkten auf jedem der beiden radiografischen Bilder, und optional zweite zu lokalisierende Bezugspunkte auf einem einzigen radiografischen Bild ausgewählt werden,
wobei das Verfahren weiter umfasst:
f) Berechnen der Koordinaten der zweiten Bezugspunkte in dem Referenzbezugssystem ausgehend von deren Koordinaten auf mindestens einem der im Schritt e) bestimmten radiografischen Bildern,
g) Korrigieren des in Schritt d) berechneten Modells, indem man Punkte des berechneten Modells, die den zweiten Bezugspunkten entsprechen, mit den Koordinaten von den im Schritt f) berechneten überdecken lässt.

8. Bildgebungsverfahren nach Anspruch 7, wobei die ersten in Schritt a) empfangenen Bilddaten gleichzeitig, insbesondere in einem selben Bezugssystem mit drei Dimensionen durch Abtasten durch Verschieben im Gleichlauf in einer selben Verschieberichtung nicht parallel zu der Aufnahmerichtung zweier radioaktiver Quellen erzeugt werden, die zwei Bündel von ionisierenden Strahlen jeweils in die beiden Aufnahmerichtungen ausgeben.

9. Bildgebungsverfahren nach einem der Ansprüche 1 oder 3 bis 8, wenn sie nicht von Anspruch 2 abhängig sind, wobei die ersten Bilddaten und die zweiten Bilddaten gleichzeitig, insbesondere in einem selben Bezugssystem mit drei Dimensionen erzeugt werden.

10. Bildgebungsverfahren nach einem der Ansprüche 1 bis 9, weiter Folgendes umfassend:
einen Übertragungsschritt, insbesondere zu einer Anzeigevorrichtung, der ausgewählten Daten aus den ersten Bilddaten, den zweiten Bilddaten, von Daten, die repräsentativ für die Koordinaten der ersten Bezugspunkte sind, von Daten, die repräsentativ für die Koordinaten jedes zweiten Bezugspunktes sind, der in Schritt e)iii) bestimmt wird, und gegebenenfalls von Daten, die repräsentativ für das im Schritt g) berechnete korrigierte Modell sind.

11. Radiografische Bildverarbeitungsvorrichtung (20), umfassend:
- Mittel (21) zum Empfangen von Bilddaten, um erste Bilddaten zu empfangen, die in einem Bezugssystem mit drei Dimensionen erzeugt werden, die repräsentativ für mindestens ein radiografisches Bild in zwei Dimensionen der internen Struktur des Gegenstandes sind, und zweiter Bilddaten, die in einem Bezugssystem mit drei Dimensionen erzeugt werden, die repräsentativ für ein Bild in drei Dimensionen einer externen Hülle des Gegenstandes sind,
- Mittel (22) zum Verarbeiten, die an die Mittel zum Empfangen angeschlossen sind, die angeordnet sind, um mindestens die Schritte c) bis e) des Verfahrens nach einem der Ansprüche 1 bis 10 auszuführen,
- optional Übertragungsmittel (23), insbesondere zu einer Anzeigevorrichtung (24).

12. Radiografische Bildgebungsvorrichtung (1) einer internen Struktur in drei Dimensionen, die Teil eines Gegenstandes ist, umfassend:
- ein erstes radiografisches Bildgebungssystem (6), das angeordnet ist, um mindestens ein radiografisches Bild in zwei Dimensionen der internen Struktur des Gegenstandes anhand mindestens einer radioaktiven Quelle (6a, 6b) aufzunehmen, und um in einem Bezugssystem mit drei Dimensionen erste Bilddaten zu erzeugen, die repräsentativ für das aufgenommene radiografische Bild sind,
- ein zweites Bildgebungssystem (15), das mindestens einen optischen Sensor (13) umfasst, der angeordnet ist, um ein Bild in drei Dimensionen einer externen Hülle des Gegenstandes aufzunehmen, und um in einem Bezugssystem mit drei Dimensionen zweite Bilddaten zu erzeugen, die repräsentativ für dieses Bild sind,
- die radiografische Bildverarbeitungsvorrichtung (20) nach Anspruch 11, die mit dem ersten und zweiten Bildgebungssystem verbunden ist, um die ersten und zweiten Bilddaten zu empfangen.

13. Computerprogramm, Programmcodeabschnitte zur Ausführung der Schritte des Bildgebungsverfahrens nach einem der Ansprüche 1 bis 10 umfassend, wenn das Programm durch eine radiografische Bildverarbeitungsvorrichtung (20) ausgeführt wird.

## Claims

1. Method for the radiographic imaging of a three-dimensional internal structure which forms part of an object located within a field of view, the method comprising:
a) receiving first image data generated in a three-dimensional reference frame of reference, representative of at least one two-dimensional radiographic image of the internal structure of the object,
b) receiving second image data generated in the reference frame of reference, representative of a three-dimensional image of an outer boundary of the object, **characterized in that** it comprises the steps of :
c) estimating from the second image data the coordinates in the reference frame of reference of first characteristic markers of the internal structure, and, optionally, the value of at least one characteristic parameter of the internal structure,
d) calculating, in the reference frame of reference, the three-dimensional shape of a model representing said internal structure from a previously known generic model of said internal structure, this generic model including points corresponding to the first markers, the calculated model being obtained by deformation of the generic model such that said calculated model observes a shape that is closest possible to an isometry of the generic model while keeping the points of the deformed generic model coincident with the first markers, and optionally by applying to the calculated model the characteristic parameter(s) of the internal structure,
e) selecting at least one second characteristic marker of the internal structure to be located on the radiographic image, and for each second marker:
i) identifying a point belonging to the model calculated in step d) corresponding to the second marker,
(ii) calculating the coordinates of a projection of this point onto the radiographic image,
iii) determining using the first image data the coordinates of the second marker on the radiographic image by searching for same in a search zone located around the coordinates of the projection.

2. Method for the radiographic imaging of a three-dimensional internal structure which forms part of an object located within a field of view, the method comprising:
a) receiving first image data generated in a three-dimensional frame of reference, representative of at least one two-dimensional radiographic image of the internal structure of the object,
b) receiving second image data generated in a further three-dimensional reference frame of reference, representative of a three-dimensional image of an outer boundary of the object,
wherein at least one of the receiving steps (a) and (b) comprises a step of transforming the image data received to express the first and second image data in the same reference frame of reference, **characterized in that** it comprises the steps of :
c) estimating from the second image data the coordinates in the reference frame of reference of first characteristic markers of the internal structure, and, optionally, the value of at least one characteristic parameter of the internal structure,
d) calculating, in the reference frame of reference, the three-dimensional shape of a model representing said internal structure from a previously known generic model of said internal structure, this generic model including points corresponding to the first markers, the calculated model being obtained by deformation of the generic model such that said calculated model observes a shape that is closest possible to an isometry of the generic model while keeping the points of the deformed generic model coincident with the first markers, and optionally by applying to the calculated model the characteristic parameter(s) of the internal structure,
e) selecting at least one second characteristic marker of the internal structure to be located on the radiographic image, and for each second marker:
i) identifying a point belonging to the model calculated in step d) corresponding to the second marker,
(ii) calculating the coordinates of a projection of this point onto the radiographic image,
iii) determining using the first image data the coordinates of the second marker on the radiographic image by searching for same in a search zone located around the coordinates of the projection.

3. Imaging method according to claim 1 or 2, wherein, the second image data received in step b) are generated by an imaging system comprising at least one optical sensor.

4. Imaging method according to any one of claims 1 to 3, wherein, step c) comprises:
(i) determining the coordinates of external markers positioned on the outer boundary in the reference frame of reference, obtained:
by calculating by means of a mathematical model, or
by processing the second image data, these external markers being marked on the outer boundary,
(ii) estimating the coordinates of the first markers in the same frame of reference using the external markers.

5. Imaging method according to any one of claims 1 to 4, wherein, the first image data received in step (a) are generated by scanning by moving at least one radioactive source in a translation direction non-parallel with an image capture direction.

6. Imaging method according to any one of the preceding claims, wherein, during step a), first image data representative of two radiographic images recorded along two non-parallel image capture directions are received.

7. Imaging method according to claim 6, wherein, during step e), the second markers are chosen among the second markers to be located on each of the two radiographic images, and optionally, from the second markers to be located on a single radiographic image,
the method further comprising:
f) calculating the coordinates of the second markers in the reference frame of reference from the coordinates thereof on at least one of the radiographic images determined in step e),
g) correcting the model calculated in step d) by coinciding the points of the calculated model corresponding to the second markers with the coordinates of those calculated in step f).

8. Imaging method according to claim 7, wherein the first image data received in step a) are generated simultaneously, by scanning, by moving in synchronism, in the same translation direction non-parallel with the image capture directions, two radioactive sources emitting two ionizing ray beams respectively in the two image capture directions.

9. Imaging method according to any one of claims 1 or 3 to 8 when they are not dependent on claim 2, wherein the first image data and the second image data are generated simultaneously, particularly in the same three-dimensional frame of reference.

10. Imaging method according to any one of claims 1 to 9 further comprising:
a step of transmission, particularly to a display device, of data chosen among the first image data, the second image data, data representative of the coordinates of the first markers, data representative of the coordinates of each second marker determined in step e)iii), and, if applicable, data representative of the corrected model calculated in step g).

11. Radiographic image processing device (20) comprising:
- means (21) for receiving image data for receiving first image data generated in a three-dimensional frame of reference, representative of at least one two-dimensional radiographic image of the internal structure of the object, and second image data generated in a three-dimension frame of reference representative of a three-dimensional image of an outer boundary of the object,
- processing means (22) connected to the receiving means arranged to carry out at least steps c) to e) of the method according to any one of claims 1 to 10,
- optionally transmission means (23), particularly to a display device (24).

12. Device (1) for the radiographic imaging of a three-dimensional internal structure which forms part of an object comprising:
- a first radiographic imaging system (6) arranged to capture at least one two-dimensional radiographic image of the internal structure of the object by means of at least one radioactive source (6a, 6b) and to generate in a three-dimensional frame of reference first image data representative of the radiographic image captured,
- a second imaging system (15) comprising at least one optical sensor (13), arranged to capture a three-dimensional image of an outer boundary of the object and to generate in a three-dimensional frame of reference second image data representative of this image,
- the radiographic image processing device (20) according to claim 11 connected to the first and second imaging systems for receiving the first and second image data.

13. Computer program comprising program code segments for executing the steps of the imaging method according to any one of claims 1 to 10, when said program is executed by a radiographic image processing device (20).
